# EUROPEAN PATENT SPECIFICATION

(11) **EP 0 648 466 B1**
(45) Date of publication and mention of the grant of the patent: **19.12.2001**
(21) Application number: 94306829.6
(22) Date of filing: 19.09.1994
(51) Int. Cl.: A61B 6/00

(54) **Radiographic imaging apparatus**
Apparat zur Erzeugung von Röntgenbildern
Appareil pour produire des images radiographiques

(30) Priority: 13.10.1993 US 135788
(43) Date of publication of application: 19.04.1995
(73) Proprietor: Marconi Medical Systems, Inc., Highland Heights, Ohio 44143 (US)
(72) Inventor: Shroy, Robert E., Willoughby, Ohio 44094 (US); Hylton, Guy F., Richmond Heights, Ohio (US)
(74) Representative: McGowan, Nigel George

(56) References cited:
- EP-A- 0 143 657
- EP-A- 0 206 156
- GB-A- 2 003 010
- US-A- 5 022 063
- US-A- 5 270 530

## Description

The present invention relates to radiographic imaging apparatus, more particularly radiographic imaging apparatus capable of operating in a fluoroscopic mode.

In a fluoroscopic/radiographic imaging system, a radiation source such as an x-ray tube directs x-rays through a patient onto an input surface of an image intensifier tube. The image intensifier tube converts a relatively large area of x-rays into a relatively small brightened visual image which corresponds to the x-ray pattern emerging from the patient. The image from the image intensifier tube is converted to a video signal by a video camera.

In a fluoroscopic/radiographic imaging system, the video signals from the video camera can be used in a variety of manners. First, the video signals can be sent directly to a viewing monitor which produces an image. In another option, the video signals can be directed to a variety of display processing components including digital acquisition systems, video tape recorders, and video disc recorders. These components digitize, enhance, and store the video signals so that they may be played back on a monitor at a later time.

The x-ray tube includes an x-ray generator power supply which provides both a voltage across the anode and cathode of the x-ray tube in kilovolts (kV) and a filament current. The filament current heats the cathode filaments boiling off electrons that are drawn by the voltage across the cathode and anode toward the anode. The electron flow, generally on the order of milliamps, is known as the tube anode current or mA. The tube anode current (mA) is controlled by adjusting the filament current. Both the tube voltage or kV and the tube anode current or mA are selectively adjustable. A timer times the selectable duration of each exposure, normally measured in seconds (s).

The contrast of the x-ray image depends significantly on the kV peak. The intensity of the x-ray dose or exposure is proportional to the product of the anode current mA and time at a given kV. This product is commonly denoted as the mAs value. In operation, the operator may set the kV value such that the resultant image has a selected contrast. In a fully manual operation, the operator commonly sets either the mAs value, or the anode current and the exposure time in order to develop an x-ray output. In theory, the resultant output, for a given kV value, should be the same for a selected mAs value regardless of whether a longer exposure time and a lower anode current or shorter exposure time and a higher anode current are selected.

In modern fluoroscopic/radiographic imaging systems, the radiographic mode and the fluoroscopic mode are used by an operator. In such systems a plurality of push-buttons are available to an operator for setting the operating parameters for various diagnostic procedures. Prior to performing a radiographic procedure, the system is commonly used in a fluoroscopic mode for positioning the patient, positioning catheters, and the like. The fluorographic mode is a very low dosage mode which produces a sufficient image for performing positioning operations. Similar to the radiographic mode, in a fluoroscopic mode, an x-ray generator power supply applies a kilovoltage (kV) to an x-ray tube and the power supply controls the tube current (mA) in the x-ray tube. This tube current and voltage control the x-ray beam which passes through a patient being examined.

When modern fluoroscopic/radiographic imaging systems are used to produce fluoroscopic images, the power supply automatically adjusts the kV and mA used to generate x-rays in the x-ray tube. In the case of pulsed fluoroscopy, the power supply control additionally sets the pulse width automatically. The kV and mA (and perhaps pulse width) are chosen to produce a constant brightness out of the image intensifier.

The power supply control may adjust kV and mA in different ways. For example, a generator operating at 100 kV and 3.0 mA which senses through a feedback arrangement that too much brightness is being produced may reduce the brightness by reducing the kV, reducing the mA, or some combination of the two. It may increase mA and decrease kV enough to give a net brightness reduction. Information on how to vary kV and mA is contained in a fluoroscopic control procedure or algorithm which is stored within the power supply control circuitry.

This fluoroscopic control procedure has a major effect on defining imaging quality. However, the kV and mA used for optimum image quality will vary according to the type of study being performed. Thus the voltage between the cathode and the anode of the x-ray tube must be chosen so as to produce x-ray photons of an energy such that the particular anatomy to be examined transmits a small but significant percentage of x-ray photons in its more absorptive structures while passing significantly more photons through its less absorptive structures. Generally, the more absorptive structures are those with a higher atomic number or higher density. Too low a peak voltage applied to the x-ray tube produces lower energy x-ray photons which are not significantly transmitted by any tissue. For a tissue which is fairly absorptive, the photon energy must be higher in order to avoid having too large a portion of the photons absorbed within the tissue. The goal is to permit an optimum percentage of the x-ray photons to pass through the tissue and to the image intensifier and video camera pick-up or film.

Therefore, when the imaging system is in a fluoroscopic mode the optimum output in large part depends upon the anatomic area which is being investigated. Further, in presently employed systems the fluoroscopic control procedure improves some image parameters at the expense of others. For example, high image contrast is achieved at lower kV's but dose reduction to the patient involves higher kV's. Systems in use today employ simple approaches to a fluoroscopic control procedure for kV and mA. In a first approach, the mA is held constant and the brightness is adjusted only by adjusting the kV. Other generators are operated to keep the mA constant at higher kV, but decrease mA as kV decreases below approximately 110 kV. In particular, the mA is decreased linearly below 110 kV at a rate that cuts the mA in half at about 80 kV.

The applicants herein have determined that existing imaging systems (as e.g. the Picker Vector and Picker Elite models, which were in public use before the priority date of this patent), maintain the necessary constant brightness by use of a single fluoroscopic control procedure irrespective of the subject, i.e. anatomic area being irradiated. Applicants have determined that the use of a single fluoroscopic control procedure cannot be optimized for all different types of fluoroscopic examinations and for all different aspects of image quality. For example, a fluoroscopic examination of an Upper Gastrointestinal study should occur at a high kV for most patients, i.e. perhaps above 100 kV. This high kV is needed to penetrate the barium used in such a study because barium is highly absorptive of x-rays produced at low kV's. On the otherhand, fluoroscopic imaging used to position a catheter in a vascular examination should use a relatively low kV, i.e. well below 100 kV. This low kV is needed because it increases contrast in the image. The catheter does not absorb as many x-rays, and would, therefore, be hard to visualize in an image without high contrast.

Another drawback of using a single fluoroscopic control procedure is that a procedure which uses higher kV will produce lower patient doses for the same detected dose. But a procedure that tends to use lower kV will produce higher image contrast. Thus, there is an inherent conflict when using a single procedure for both low dose applications and in high contrast applications.

It is an object of the present invention to provide a radiographic imaging apparatus wherein the above described problems are alleviated.

According to the present invention there is provided a radiographic imaging apparatus capable of operation in a fluoroscopic mode comprising: an x-ray tube for generating a beam of radiation which in use of the apparatus is directed through a subject and impinges on a radiation detecting means; an operator input means for inputting commands to control operation of the x-ray tube and hence the imaging apparatus; a fluoroscopic control procedure storage means for storing at least two different fluoroscopic control procedures, each said procedure defining how the kV and mA applied to the x-ray tube (A) will vary relative to one another in use of the apparatus; and a fluoroscopic control procedure selecting means for selecting, when the apparatus is operating in the fluoroscopic mode, a particular fluoroscopic control procedure from among the stored fluoroscopic control procedures, said selecting means selecting the particular control procedure in dependence on commands input by the operator on said operator input means.

Preferably the input means comprises a plurality of selectors for inputting commands to control operation of the x-ray tube. The input selectors typically enable control of the operation of the x-ray tube in dependence on at least one of: anatomic area of the subject to be studied; the required study type; the desired quality of the image of the subject to be obtained and; the radiation dose to be applied to the subject.

One advantage of the present invention is that it provides more than one fluoroscopic control procedure which can be used when the x-ray device is in a fluoroscopic mode.

Another advantage of the present invention is that it provides an improved x-ray output in the fluoroscopic mode for selected investigations.

Another advantage of the present invention is that it allows selection of a fluoroscopic control procedure that will give an acceptable image and also reduce the dose of radiation received by patients.

One radiographic imaging apparatus in accordance with the invention will now be described, by way of example, with reference to the accompanying drawings in which;
Figure 1 is a diagrammatic illustration of the radiographic imaging apparatus;
Figure 2 is a flow chart showing the mode selection procedures used in the apparatus of Figure 1;
Figures 3-6 are graphs of kV versus mA for various selectable fluorographic control procedures of the present invention.

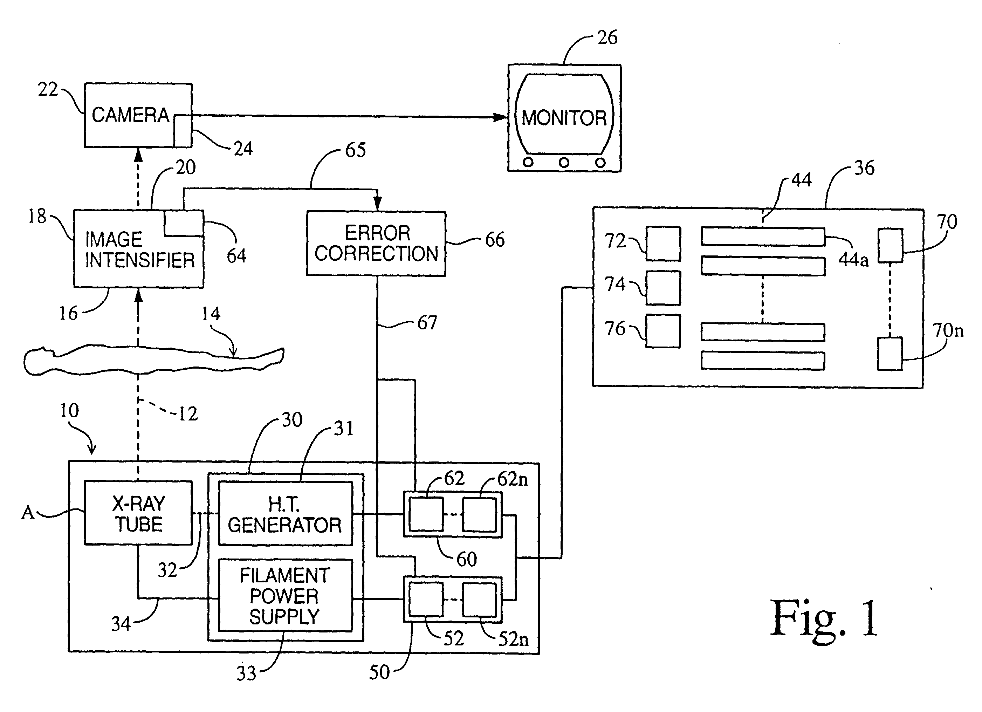

Referring to Figure 1, the apparatus includes a source of penetrating radiation 10 directed along a path 12. The path causes the radiation to pass through a patient 14 and to impinge upon an input face 16 of an image intensifier tube 18. The x-rays from the source which have passed through the patient 14 emerge and impinge upon the intensifier tube's input face 16 in a unique radiation pattern. The image tube 18 converts the relatively large area of x-rays into a relatively small bright visual image corresponding to the x-ray pattern which emerged from the patient, at the image tube's output face 20.

A video camera 22 receives light output from the output face 20 of image intensifier tube 18. The camera 22 produces analog video signals, including synchronization information, which represent the viewed image. The video signals, which are low level signals, are processed in an amplifier means or circuit 24 which may be part of the camera 22. The amplifier 24 boosts the low level video signals up to more readily usable values and selectively filters the amplified signals removing unused bandwidths. The video and synchronization signals can then be transmitted directly to a monitor 26 for display. Alternatively, a video signal processing subsystem (not shown) may enhance or process the signals before they are sent to the monitor 26.

In the fluoroscopic/radiographic imaging system of Figure 1, the source of penetrating radiation 10 includes power supply system 30 which applies a kilovoltage (kV) 32 to an x-ray tube A and controls the emission current (mA) 34 in the x-ray tube A. This current (mA) and voltage (kV) control the output of the x-ray beam 12 which passes through the patient 14 being examined. A high voltage or high tension generator 31 generates the selected tube voltage and applies the kV 32 across the cathode and anode of the x-ray tube. Analogously, a current power supply 33 controls the emission current (mA) 34.

Returning attention to the source of penetrating radiation 10, a radiographic control procedure storage 50 stores a plurality of differing radiographic control procedures **52-52**_{**n**}**.** When the operation is in the radiographic mode and the operator has picked a particular anatomic selector from the plurality of selectors **44** arranged on control panel **36**, one of the stored radiographic control procedures **52-52**_{**n**} will be selected to control operation of the imaging system. In this manner, the appropriate investigation of the subject **14** will irradiated at an appropriate intensity. It is to be appreciated that the selectors **44** may be used to select a desired anatomic area of the subject, a particular type of study, a procedure for optimizing various aspects of image quality, a procedure to control patient dose and/or various combinations of the above features.

A fluoroscopic control procedure storage **60** stores at least two different fluoroscopic control procedures **62-62**_{**n**}**.** When one of the anatomic selectors **44** is chosen by an operator, and the operator has further selected the fluoroscopic mode then one of the stored fluoroscopic control procedures **62-62**_{**n**}**,** which will produce the most optimal image is selected and used to control the output from the source of penetrating radiation **10.** The fluoroscopic control procedure storage **60** may be located as a separate storage area, as part of the source of penetrating radiation **10** or as part of an intelligent control panel.

The image intensifier **18** includes a sensor **64** which will sense the intensity or brightness of the produced image. The sensor means **64** provides a feedback signal **65** to an error correction means **66.** The error correction means will compare the anticipated brightness or intensity with the actual received value and provide an error adjustment signal **67** for use by one of the fluoroscopic control procedures **62-62**_{**n**}. Wherein these procedures will integrate the error signal in the control operation. Alternatively, the error signal may be passed directly to the power supply system **30** to be used by the high tension generator and/or the current power supply to adjust the x-ray output.

An important concept of the present invention is based on the realization that one control procedure cannot produce optimum image quality for all types of studies. Also, one control procedure cannot optimize all aspects of image quality. The present invention, thus, is directed to providing a generator with a variety of different control procedures or algorithms. Each procedure used is optimized for use in a specific area of investigation. In this manner, a fluorographic control procedure can be selected which matches the requirements of the desired investigations, such as selection of an anatomic area.

To avoid burdening the operator with an extra chore of selecting a particular fluoroscopic control procedure, the procedures are programmed to be responsive to each of the anatomical control procedures that may be selected by the operator. Therefore, for instance when an Upper Gastrointestinal selector **44a** is pressed, a typical set of radiographic factors for such a study is automatically loaded to control the x-ray tube **A** output. In the present embodiment, when the system is in the fluoroscopic mode and the Upper Gastrointestinal selector **44a** is chosen a fluorographic control procedure stored in storage **60**, appropriate for this investigation, is used to control kV, mA and pulse width (if pulsed fluoro is used) by which the x-ray tube output is controlled. In fluoroscopy, using a signal representing a particular anatomic area, an appropriate fluoroscopic control procedure **62-62**_{**n**} is retrieved and used to control the power supply **30**. It is to be appreciated that the system is further arranged to select an appropriate radiographic control procedure **52-52**_{**n**} when the imaging system is in the radiographic mode.

An alternative embodiment of the present invention would be to have different fluoroscopic control procedures stored in software on different integrated circuits and selected integrated circuits could be installed into the system at different sites. Yet another embodiment would be to select the fluoroscopic control procedures using labeled mechanical switches **70-70**_{**n**} as shown in FIGURE 1.

In another feature of the present invention, the operator may activate an override selector **72** to allow for manual control of mA and kV by switches **74** and **76.** In this mode the fluoroscopic control procedures are by-passed and mA and kV output are controlled directly by the operator.

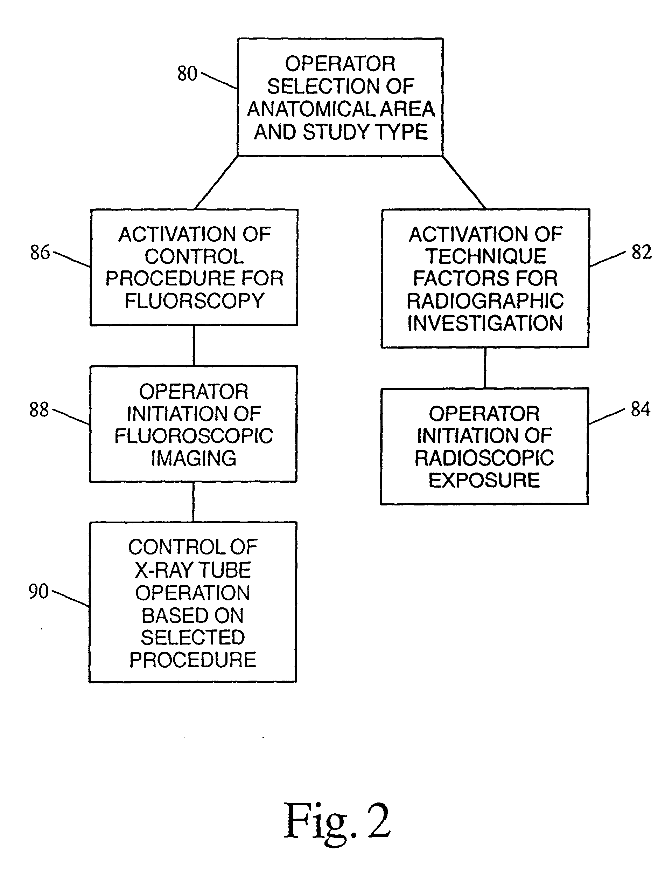

FIGURE 2 sets forth a flow chart showing selection of particular fluoroscopic control procedures according to the present invention. An operator will initially select a desired anatomic area and type of study, **80**. In known imaging control systems such as MTX 340, MTX 360, MTX 380 and MTX 3100, from Picker International 24 different anatomic areas of the human body may be selectively chosen for investigation. Based on these selections, radiographic parameters appropriate for these selections will be entered in a known manner into generator radiographic controls, **82**. As more particularly directed to the present invention, the fluorographic control procedure appropriate for the selected study will be entered into generator fluoroscopic controls, **86**.

After control parameters and procedures are entered into generator controls, the operator will usually initiate fluoroscopic imaging, **88**, to position the imaging system with respect to the anatomy to be imaged. Thereafter, the examination includes various operations of radiographic imaging, **84** interspersed with further fluoroscopic imaging. In the case of fluoroscopic imaging, the appropriate fluoroscopic control procedure will be used to provide the desired kV, mA and (in the case of pulsed fluoroscopy) pulse width for control of the x-ray tube output, **90**.

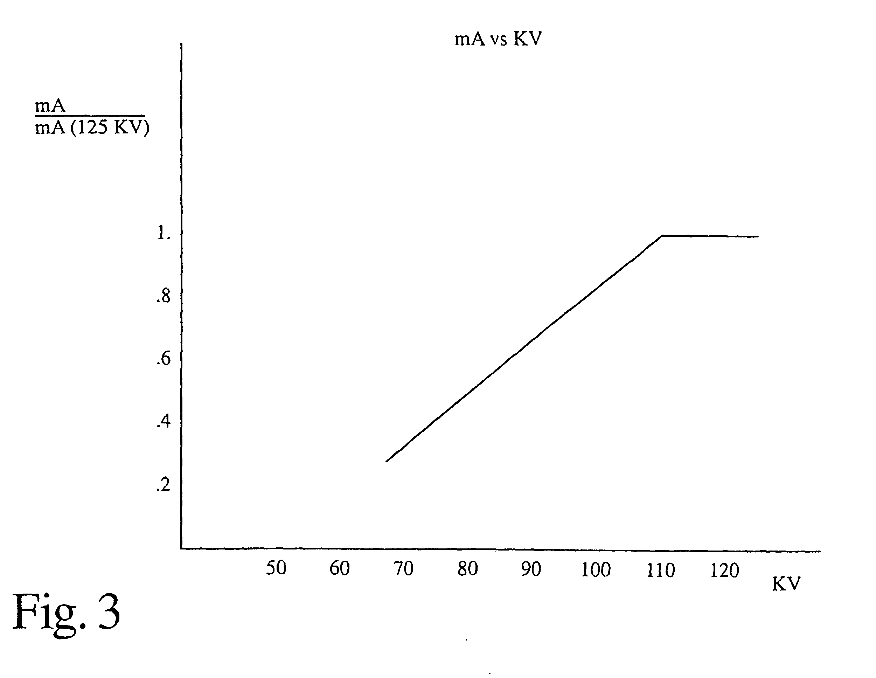

FIGURES 3-6 use kV versus mA graphs to detail outputs obtained by fluoroscopic control procedures which may be implemented in the present invention, as applied to continuous fluoroscopy.

It is to be appreciated that there will not be a single optimum fluoroscopic control procedure resulting in a single kV/mA curve for all investigations because the best procedure will depend on the type of study being performed. The best procedure will also depend on what image parameter is to optimized. Therefore, discussed below are several possible applications and their kV/mA curves.

First, in a study using barium the kV/mA curve commonly used in continuous fluoro is one that has been used in other generators. It was designed for barium imaging and is shown in FIGURE 3 as a solid line. This curve decreases mA when kV decreases below about 110 kV tending to keep the kV at higher values. The higher kV are needed to move through dense accumulations of barium; if kV were too low, the image would only show black (areas with barium) and white (areas with no barium).

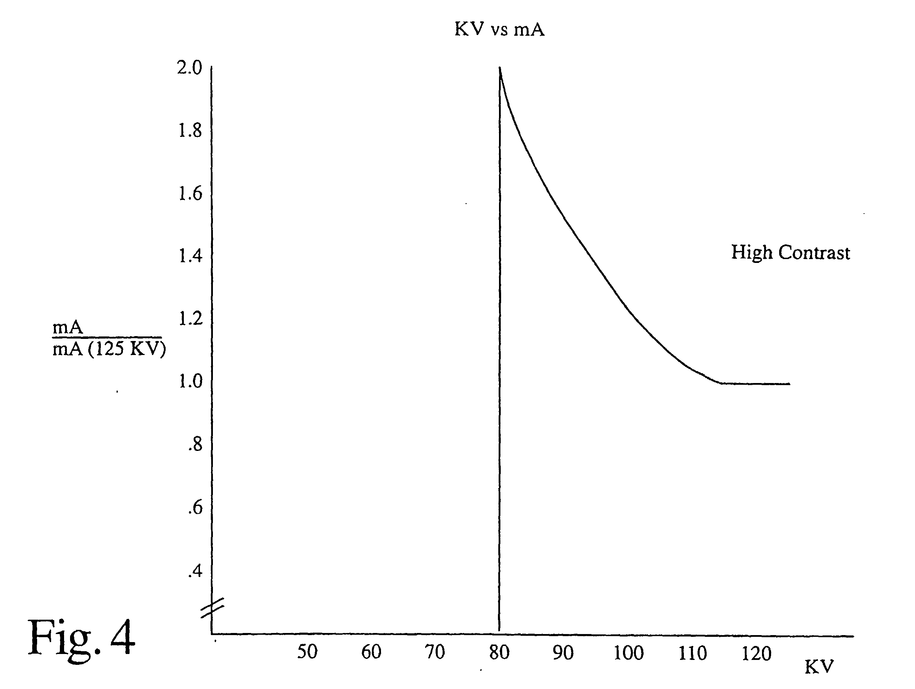

Another fluoroscopic control procedure is one which provides high contrast in soft tissue. As shown by FIGURE 4, high contrast comes at lower kV's. Thus, high contrast comes with a kV/mA curve that tries to stay low in kV, but will not go below 80 kV. FIGURE 4, therefore, is an example of a curve that obtains relatively higher contrast.

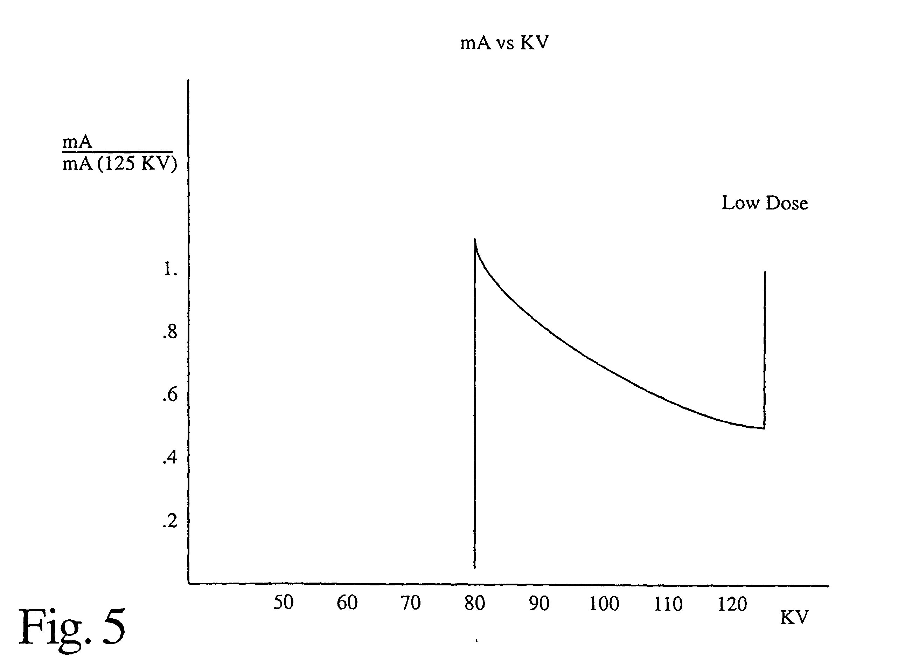

There will be other procedures which will provide low dose imaging. As detailed in FIGURE 5, low dose operation is generally achieved at higher kV's, although the dose reduction is smaller at very high kV because of reduced efficiency of the image intensifier. This is the opposite requirement as that discussed above in connection with FIGURE 4 regarding high contrast.

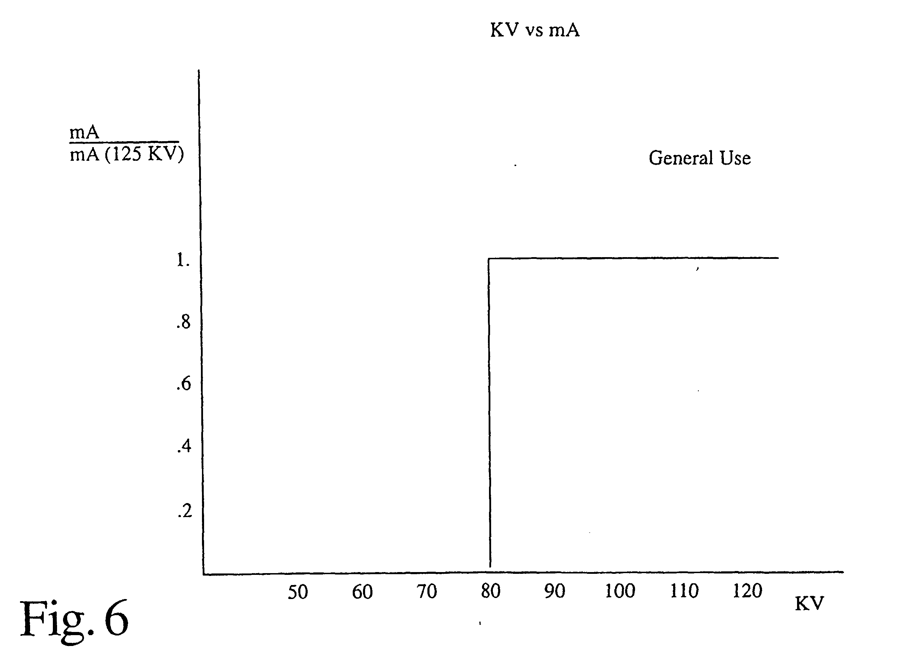

There is also a general use fluoroscopic control procedure when a curve of high-contrast or low-dose may be too extreme. In this case, an intermediate procedure will be desired. A curve of one such procedure is the straight line curve shown in FIGURE 6.

It is further possible to optimize control procedures for many other types of studies, including pediatric studies.

The above discussed Figures 3-6 provide examples of tailoring the fluoroscopic control procedurers to particular applications. These particularized procedures are then linked to specific areas of anatomic investigations and specific types of studies. Then when a specific anatomic area is investigated in a fluoroscopic mode a tailored fluoroscopic control procedure is called to provide an optimized image of that area.

It is also to be appreciated that though the present invention has generally been described in a medical area, the provision of fluoroscopic imaging in non-medical situations such as industrial situations is also envisaged.

## Claims

1. A radiographic imaging apparatus capable of operation in a fluoroscopic mode comprising: an x-ray tube (A) for generating a beam of radiation which in use of the apparatus is directed through a subject (14) and impinges on a radiation detecting means (16); an operator input means (36) for inputting commands to control operation of the x-ray tube (A) and hence the imaging apparatus; a fluoroscopic control procedure storage means (60) for storing at least two different fluoroscopic control procedures (62), each said procedure defining how the kV and mA applied to the x-ray tube (A) will vary relative to one another in use of the apparatus; and a fluoroscopic control procedure selecting means for selecting, when the apparatus is operating in the fluoroscopic mode, a particular fluoroscopic control procedure (62) from among the stored fluoroscopic control procedures (62), said selecting means selecting the particular control procedure (62) in dependence on commands input by the operator on said operator input means (36).

2. An apparatus according to Claim 1 wherein said input means (36) comprises a plurality of selectors (44, 70, 72, 74, 76) for inputting commands to control operation of the x-ray tube (A).

3. An apparatus according to Claim 2 wherein the input selectors include selectors (44) to enable control of the operation of the x-ray tube (A) in dependence on at least one of: the anatomic area of the subject (14) to be studied; the required study type; the desired quality of the image of the subject (14) to be obtained; and the radiation dose to be applied to the subject (14).

4. An apparatus according to any preceding claim wherein the input means includes override means (72, 74, 76)for direct control of kV and mA by the operator.

5. An apparatus according to any preceding claim wherein the fluoroscopic control procedure selecting means effects control of pulse width in the case of the apparatus having a pulsed fluoroscopy mode.

6. An apparatus according to any preceding claim wherein the fluoroscopic control procedure selecting means effects decrease of mA applied to the x-ray tube (A) when kV decreases below a selected value.

7. An apparatus according to any preceding claim wherein the fluoroscopic control procedure selecting means enables use of a relatively low kV radiation beam to obtain a high contrast image of soft tissue.

8. An apparatus according to any preceding claim wherein the fluoroscopic control procedure selecting means enables provision of a low radiation dose to the subject (14) by increasing kV and lowering mA.

9. An apparatus according to any preceding claim wherein the fluoroscopic control procedure selecting means includes means (70) for manual setting of fluoroscopic control procedures.

## Patentansprüche

1. Vorrichtung zum Erzeugen von Röntgenbildern, die in einem Fluoroskopiemodus arbeiten kann, umfassend: eine Röntgenröhre (A) zum Erzeugen eines Strahlungsstrahls, welcher beim Betrieb der Vorrichtung durch ein Subjekt (14) hindurch gerichtet ist und auf eine Strahlungsdetektoreinrichtung (16) auftrifft; eine Bedienereingabeeinrichtung (36) zum Eingeben von Befehlen zur Steuerung des Betriebs der Röntgenröhre (A) und somit der Bildgebungsvorrichtung; eine Speichereinrichtung (60) für Fluoroskopiesteuerprozeduren, zum Speichern wenigstens zweier unterschiedlicher Fluoroskopiesteuerprozeduren (62), wobei jede solche Prozedur definiert, wie sich die an die Röntgenröhre (A) angelegten kV und mA beim Betrieb der Vorrichtung in Bezug aufeinander ändern werden; und eine Fluoroskopiesteuerprozedur-Auswahleinrichtung zum Auswählen einer bestimmten Fluoroskopiesteuerprozedur (62) aus den gespeicherten Fluoroskopiesteuerprozeduren (62), wenn die Vorrichtung im Fluoroskopiemodus arbeitet, wobei die Auswahleinrichtung die bestimmte Fluoroskopiesteuerprozedur in Abhängigkeit von den durch den Bediener an der Bedienereingabeeinrichtung (36) eingegebenen Befehlen auswählt.

2. Vorrichtung nach Anspruch 1, bei welcher die Eingabeeinrichtung (36) mehrere Wahlschalter (44, 70, 72, 74, 76) zum Eingeben von Befehlen aufweist, um den Betrieb der Röntgenröhre (A) zu steuern.

3. Vorrichtung nach Anspruch 2, bei welcher die Wahleingabeschalter Wahlschalter (44) aufweisen, um eine Steuerung des Betriebs der Röntgenröhre (A) in Abhängigkeit von wenigstens einem folgender Merkmale freizugeben: dem zu untersuchenden anatomischen Bereich des Subjektes (14); der Art der erforderlichen Untersuchung; der gewünschten Qualität des zu erzielenden Bildes des Subjektes (14); und der an dem Subjekt (14) anzuwendenden Strahlendosis.

4. Vorrichtung nach einem der vorhergehenden Ansprüche, bei welcher die Eingabeeinrichtung Umgehungsschalter (72, 74, 76) aufweist, um die Steuerung der kV und mA durch den Bediener auszuführen.

5. Vorrichtung nach einem der vorhergehenden Ansprüche, bei welcher durch die Auswahleinrichtung für Fluoroskopiesteuerprozeduren im Falle, dass die Vorrichtung einen gepulsten Fluoroskopiemodus beinhaltet, eine Steuerung der Pulsbreite erfolgt.

6. Vorrichtung nach einem der vorhergehenden Ansprüche, bei welcher durch die Auswahleinrichtung für Fluoroskopiesteuerprozeduren die Verringerung der an die Röntgenröhre (A) angelegten mA erfolgt, wenn die kV unter einen gewählten Wert absinken.

7. Vorrichtung nach einem der vorhergehenden Ansprüche, bei welcher die Auswahleinrichtung für Fluoroskopiesteuerprozeduren die Verwendung eines Strahlungsstrahls mit relativ wenig kV ermöglicht, um ein Bild von Weichteilgewebe mit hohem Kontrast zu erhalten.

8. Vorrichtung nach einem der vorhergehenden Ansprüche, bei welcher die Auswahleinrichtung für Fluoroskopiesteuerprozeduren die Abgabe einer geringen Strahlendosis auf das Subjekt (14) ermöglicht, indem die kV erhöht und die mA verringert werden.

9. Vorrichtung nach einem der vorhergehenden Ansprüche, bei welcher die Auswahleinrichtung für Fluoroskopiesteuerprozeduren eine Einrichtung (70) zum manuellen Einstellen der Fluoroskopiesteuerprozeduren beinhaltet.

## Revendications

1. Appareil d'imagerie radiographique capable de fonctionner en mode fluoroscopique, comprenant : un tube à rayons X (A) pour générer un faisceau de rayonnement qui, pendant l'utilisation de l'appareil, est dirigé à travers un sujet (14) et parvient à des moyens de détection de rayonnement (16); des moyens d'entrée d'opérateur (36) pour entrer des commandes pour commander le fonctionnement du tube à rayons X (A) et donc l'appareil d'imagerie; des moyens de stockage de procédures de commande fluoroscopique (60) pour stocker au moins deux procédures de commande fluoroscopique différentes (62), chacune desdites procédures définissant comment les kV et mA appliqués au tube à rayons X (A) varieront l'un par rapport à l'autre en cours d'utilisation de l'appareil; et des moyens de sélection de procédure de commande fluoroscopique pour sélectionner, quand l'appareil fonctionne en mode fluoroscopique, une procédure de commande fluoroscopique (62) parmi les procédures de commande fluoroscopique (62) stockées, lesdits moyens de sélection sélectionnant la procédure de commande (62) particulière en fonction de commandes entrées par l'opérateur sur lesdits moyens d'entrée de l'opérateur (36).

2. Appareil selon la revendication 1, dans lequel lesdits moyens d'entrée (36) comprennent une pluralité de sélecteurs (44, 70, 72, 74, 76) pour entrer des commandes pour commander le fonctionnement du tube à rayons X (A).

3. Appareil selon la revendication 2, dans lequel les sélecteurs d'entrée comprennent des sélecteurs (44) pour permettre la commande du fonctionnement du tube à rayons X (A) en fonction d'au moins un élément parmi : une zone anatomique du sujet (14) à étudier; le type d'étude nécessaire; la qualité souhaitée de l'image du sujet (14) à obtenir; et la dose de rayonnement à appliquer au sujet (14).

4. Appareil selon l'une quelconque des revendications précédentes, dans lequel les moyens d'entrée comprennent des moyens de commande manuelle (72, 74, 76) pour une commande directe des valeurs kV et mA par l'opérateur.

5. Appareil selon l'une quelconque des revendications précédentes, dans lequel les moyens de sélection de procédure de commande fluoroscopique effectuent la commande de la largeur d'impulsion au cas où l'appareil aurait un mode de fluoroscopie pulsée.

6. Appareil selon l'une quelconque des revendications précédentes, dans lequel les moyens de sélection de procédure de commande fluoroscopique effectuent une diminution du mA appliqué au tube à rayons X (A) quand le kV descend au-dessous d'une valeur sélectionnée.

7. Appareil selon l'une quelconque des revendications précédentes, dans lequel les moyens de sélection de procédure de commande fluoroscopique permettent l'utilisation d'un faisceau de rayonnement d'un kV relativement bas pour obtenir une image à contraste élevé d'un tissu mou.

8. Appareil selon l'une quelconque des revendications précédentes, dans lequel les moyens de sélection de procédure de commande fluoroscopique permettent la fourniture d'une dose de rayonnement faible au sujet (14) en augmentant le kV et en diminuant le mA.

9. Appareil selon l'une quelconque des revendications précédentes, dans lequel les moyens de sélection de procédure de commande fluoroscopique comprennent des moyens (70) pour définir manuellement des procédures de commande fluoroscopique.
